# EUROPÄISCHE PATENTSCHRIFT

(11) **EP 1 931 466 B1**
(45) Veröffentlichungstag und Bekanntmachung des Hinweises auf die Patenterteilung: **14.11.2012**
(21) Anmeldenummer: 06793698.9
(22) Anmeldetag: 21.09.2006
(51) Int. Cl.: B01J 21/12, B01J 21/04, B01J 35/10, C07C 211/04, C07C 209/16

(54) **FORMKÖRPER ENTHALTEND EIN ALUMOSILIKAT UND ALUMINIUMOXID UND VERFAHREN ZUR KONTINUIERLICHEN HERSTELLUNG VON METHYLAMINEN**
MOULDED BODY CONTAINING ALUMINOSILICATE AND ALUMINUM OXIDE AND METHOD FOR CONTINUOUSLY PRODUCING METHYLAMINES
CORPS MOULE CONTENANT UN SILICATE D'ALUMINIUM ET UN OXYDE D'ALUMINIUM ET PROCEDE POUR LA PRODUCTION EN CONTINU DE METHYLAMINES

(30) Priorität: 29.09.2005 DE 102005046815; 18.10.2005 DE 102005049746; 01.06.2006 EP 06114868
(43) Veröffentlichungstag der Anmeldung: 18.06.2008
(73) Patentinhaber: BASF SE, 67056 Ludwigshafen (DE)
(72) Erfinder: BOSCH, Marco, 68159 Mannheim (DE); RÖTTGER, Roderich, 68165 Mannheim (DE); EBERHARDT, Jan, 68167 Mannheim (DE); KRUG, Thomas, 67550 Worms (DE); WEBER, Theodor, 67063 Ludwigshafen (DE); ROSS, Karl-Heinz, 67269 Grünstadt (DE); JULIUS, Manfred, 67117 Limburgerhof (DE)
(86) Internationale Anmeldenummer: PCT/EP2006/066575
(87) Internationale Veröffentlichungsnummer: WO 2007/036478

(56) Entgegenhaltungen:
- WO-A-02/055192
- WO-A-2004/108280
- WO-A-2005/123658
- WO-A-2006/032782
- DD-A1- 108 275
- DATABASE WPI Week 199341 Derwent Publications Ltd., London, GB; AN 1993-326795 XP002412361 & SU 1 766 499 A1 (LENGD PETROCHEM PROCESSES DEV ASSOC) 7. Oktober 1992 (1992-10-07) in der Anmeldung erwähnt

## Beschreibung

Die vorliegende Erfindung betrifft einen Formkörper enthaltend ein Alumosilikat und Aluminiumoxid, ein Verfahren zu seiner Herstellung und ein Verfahren zur kontinuierlichen Herstellung von Methylaminen durch Umsetzung von Methanol und/oder Dimethylether mit Ammoniak.

Monomethylamin (MMA) ist ein Zwischenprodukt, welches in der Synthese von Pharmazeutika (z.B. Theophyllin), Pestiziden (Carbaryl, Metham Natrium, Carbofuran), Tensiden, photographischen Entwicklern, Sprengstoffen und Lösungsmitteln wie N-Methyl-2-pyrrolidon (NMP) eingesetzt wird.

Dimethylamin (DMA) ist ebenfalls ein synthetisches Zwischenprodukt. Beispiele für Produkte auf Dimethylamin-Basis sind Fungizide und Vulkanisationsbeschleuniger (Zink-bis-Dimethyldithiocarbamat) (Ziram), Tetramethyl-thioperoxydikohlensäurediamid (TMTD), Tetramethyl-thiokohlensäure-diamid (MTMT), das Treibmittel 1,1-Dimethylhydrazin, verschiedene Pharmazeutika, Monomere wie z.B. Dimethylaminoethylmethacrylat, Lösungsmittel (N,N-Dimethylformamid, N,N-Dimethylacetamid), Katalysatoren [z. B. 2,4,6-Bis[(dimethylamino)methyl]phenol (DMP 30)], das Insektizid Dimefax, Tenside und Ionenaustauschharze.

Trimethylamin (TMA) wird bei der Herstellung von Cholinsalzen, kationischen Stärken, Desinfektionsmitteln, Flotationsmitteln, Süßstoffen und Ionenaustauschharzen eingesetzt.

Die Synthese von Monomethylamin (MMA), Dimethylamin (DMA) und Trimethylamin (TMA) erfolgt aus Ammoniak und Methanol in der Gasphase z.B. an amorphem Alumina, oder Silica-Alumina (Mischformen von Aluminium- und Siliziumoxid) bei Drücken von 10 bis 50 bar. Bei der Anwendung von höheren Temperaturen (350 bis 475°C) stellt sich an diesen heterogenen Katalysatoren das thermodynamische Gleichgewicht ein oder wird näherungsweise erreicht, wenn die Verweilzeit im Reaktor bei gegebenem Druck und gegebener Zulauftemperatur hinreichend ist (siehe: Catalysis Today, 1997, 37, Seiten 71 - 102).

Für die Alkylierung von Ammoniak, Monomethylamin (MMA) und/oder Dimethylamin (DMA) mit Methanol (= Aminierungsreaktion) gilt, dass die Reaktionsgeschwindigkeit in der Reihe NH₃ < MMA < DMA zunimmt. Dementsprechend liegt der Anteil an TMA an den amorphen "Gleichgewichtskatalysatoren" im geraden Durchgang, bezogen auf die Summe von MMA, DMA und TMA, zwischen 35 und 75 Gewichtsprozent. Die Produktverteilung ist abhängig von der Temperatur und vom N/C-Verhältnis (siehe: Ind. Eng. Chem. Res. 2004, 43, Seiten 5123 - 5132).

Der weltweite Verbrauch an Trimethylamin liegt bezogen auf die Gesamtmenge der Methylamine bei weniger als 20 Gewichtsprozent (siehe: Ullmann's Encyclopedia of Industrial Chemistry, 5. Ausgabe, A16, Seiten 535 - 541). Es ist daher notwendig, den Anteil an DMA und MMA im Reaktionsgemisch zu erhöhen.
Dies gelingt durch Rückführung von TMA und Ammoniak auf dem Katalysator, in der Synthese wird das TMA in ein Gemisch aus MMA, DMA und TMA umgewandelt (= Transalkylierungsreaktion). Das Verfahren zur Herstellung von Methylaminen aus Methanol und Ammoniak, wobei die Aminierungs- und Transalkylierungsreaktion kombiniert wird, wird als Leonard Prozess bezeichnet (siehe: US 3,387,032, "Alkylamines" PEP Report No. 138, 1981, (SRI International, Menlo Park, California) und Hydrocarbon Process 1979, 58, Seiten 194ff). Durch die Rückführung kann der Produktmix je nach Bedarf eingestellt werden; ein typisches Beispiel ist ein Gemisch bestehend aus 34 Gew.-% MMA, 46 Gew.-% DMA und 20 Gew.-% TMA ("Alkylamines" PEP Report No. 138, 1981).

In US 1,875,747 (Martin et al.) wird die Herstellung von Methylaminen aus NH₃ und MeOH an Alumosilikatkatalysatoren bei Temperaturen zwischen 300 und 500°C beschrieben. Das Alumosilikat kann als synthetischer Feststoff oder in Form von Tonerde eingesetzt werden.

In EP-A-64 380 (DuPont) wird ein Katalysator beschrieben welcher durch die Behandlung eines Alumosilikats mit den Hydroxidsalzen des Natriums, Kaliums, Lithiums, Bariums oder Strontiums erhalten wird, wobei der Anteil an Na, K, Li, Ba oder Sr zwischen 0,1 und 6 Gew.-% liegt. Die Verwendung des Katalysators für die Herstellung von Methylaminen aus Methanol und Ammoniak, wobei der Anteil an Monomethylamin (MMA) durch eine Zunahme des Alkali- bzw. Erdalkalianteils eingestellt werden kann, wird ebenfalls erwähnt.

In DD-A-266 096 (VEB Leuna) wird ein Verfahren zur Herstellung von Methylaminen an einem SiO₂-Al₂O₃-Dehydratisierkatalysator beschrieben, wobei ein Al₂O₃- oder Al₂O₃/SiO₂-Träger im Wirbelbett mit einer salpetersäurehaltigen Suspension von feingemahlenem Böhmit und Kaolin in Wasser besprüht wird und der kugelförmige Katalysator nach thermischer Aktivierung bei 400 bis 600°C ein Schüttgewicht von 0,5 bis 0,6 kg/l, einen Al₂O₃-Gehalt von 70 bis 80 % sowie ein Porenvolumen von 0,75 bis 0,85 ml/g aufweist, wobei das Porenvolumen im Bereich der Poren mit Durchmesser unterhalb von 15 nm mindestens 0,3 ml/g und im Bereich der Poren mit Durchmesser oberhalb von 15 nm mindestens 0,4 ml/g und davon wiederum im Bereich der Poren mit einem Durchmesser oberhalb 100 nm mindestens 0,2 ml/g aufweisen muss. Die erfindungsgemäßen Katalysatoren haben angeblich eine 20 % längere Standzeit und weisen nach Ausbau weniger kohlenstoffhaltige Ablagerungen und zerstörte Formkörper auf. Der Anteil von Al₂O₃ zwischen 70 und 80 % entspricht einem molaren Al/Si-Verhältnis zwischen 2,7 und 4,7.

In DD-A-149 213 (VEB Leuna) wird ein Verfahren zur Herstellung von Methylaminen an dehydratisierend wirkenden Katalysatoren, die aktives Aluminiumoxid enthalten, offenbart, wobei ein auf der Basis von Kaolin und Pseudoböhmit hergestellter Katalysator verwendet wird, der neben Aluminiumoxid 12 bis 18 Gew.-% Siliciumdioxid enthält, dessen Gesamtporenvolumen größer als 0,5 ml/g ist, wobei der Anteil der Poren mit einem Durchmesser kleiner als 4 nm mindestens 30 % und größer als 15 nm höchstens 10 % beträgt und seine Korngröße bzw. Wandstärke kleiner 4 mm ist. Der Katalysator konnte laut Angaben 13 Monate ohne signifikante Deaktivierung [U(MeOH) > 99 %] gefahren werden, die Ablagerung auf dem Ausbaukatalysator enthielt ca. 1,2 Gew.-% Kohlenstoff. Der Anteil von SiO₂ zwischen 12 und 18 % entspricht einem molaren Al/Si-Verhältnis zwischen 5,4 und 8,6.

In EP-A-62 428 (= US 4,370,503) wird die Verwendung eines Katalysators bestehend aus 88 bis 99 Gew.-% Alumina und 1 bis 13 Gew.-% Silica für die Herstellung von Methylaminen aus Methanol oder Dimethylether und Ammoniak beschrieben. Die Gewichtsverteilungen an Silica und Alumina entsprechen einem molaren Al/Si-Verhältnis von 7,9 bis 116,7. Der Katalysator liegt üblicherweise als Tablette mit einem Durchmesser und/oder einer Länge von 3 bis 13 mm vor. Das Porenvolumen der Tabletten liegt zwischen 0,2 und 0,8 ml/g, die BET-Oberfläche zwischen 100 und 250 m²/g.

In DD-A-108 275 (Becker et al.) wird ein Verfahren zur Herstellung von Methylaminen an fest angeordneten Katalysatoren, bestehend aus Aluminiumoxid und/oder Alumosilikaten, beschrieben, wobei der Katalysator in Form eines Hohlstranges verwendet wird, dessen Gesamtdurchmesser 3 bis 10 mm und dessen Hohlraumdurchmesser 1 bis 5 mm betragen, mit mindestens 30 % Poren mit einem Durchmesser größer als 100 Ä, einer Oberfläche von mindestens 130 m²/g und einer Azidität von höchstens 2,0 x 10-5 mol NH₃/g. Im Vergleich zum Vollstrang wurden angeblich bei Verwendung des Hohlstranges ein um 41 % geringerer Druckverlust gemessen, weniger Nebenkomponenten gebildet, konnte die Ablagerung wasserstoffarmer Verbindungen reduziert und die Standzeit des Katalysators um.30 % erhöht werden.

In SU-A1-1766 499 (Chem. Abstr. 120: 57149) wird die Herstellung eines Silica-Alumina-Katalysators beschrieben. Die Besserstellung gegenüber dem Stand der Technik besteht gemäß der Beschreibung in der Verwendung von Al(OH)₃ statt Pseudoböhmit, Senkung des Kaolin zu Aluminiumhydroxid - Massenverhältnisses von 50 auf 10, Zugabe von Rückgemahlenem bei der Katalysatorherstellung und Peptisierung des Gemisches vor der Extrusion mit HNO₃, gefolgt von einer Behandlung mit einer ammoniakalischen Lösung. Durch die Maßnahmen kann angeblich die Aktivität gesteigert und die mechanische Eigenschaft der Formkörper verbessert werden.

In SU-A1-1766 500 (Chem. Abstr. 120: 57148) wird aufbauend auf SU-A1-1766 499 die vorteilhafte Verwendung von Ameisensäure statt Salpetersäure zur Peptisierung der Extrusionsmasse beschrieben.

In DE-A-1 543 731 (Leonard) wird ein Verfahren zur Herstellung von Aminen durch Umsetzung von Ammoniak mit Alkoholen, Äthern und Mischungen davon in der Dampfphase und in Gegenwart von Katalysatoren gelehrt, wobei der Katalysator eine Silicagelbasis aufweist, auf welche aktives Aluminiumoxid und Spuren von Metallsalzvermittlern aufgetragen sind. Der Katalysator wird vor Benutzung durch eine Behandlung mit Wasserdampf bei 1 - 50 Atmosphären teilweise deaktiviert. Der Katalysator enthält üblicherweise zwischen 12 und 13 Gew.-% Al₂O₃. Durch die Dampfbehandlung wird die Gesamtoberfläche des Katalysators auf 90 ± 20 m²/g reduziert und das Porenvolumen auf 0,34 ± 0,10 ml/g und der Porendurchmesser auf 74 ± 10 Ä eingestellt. Durch die Deaktivierung des Alumosilikats und die Auftragung von Silber-, Rhenium-, Molybdän- und Cobaltsalzen konnte angeblich der Anteil an DMA im Reaktionsgemisch von 32 bis 35 Gew.-% auf > 50 Gew.-% angehoben werden.

Nach Angaben des o.g. PEP-Reports von 1981, der über die Herstellung von Alkylamine, speziell Methylamine, berichtet, beträgt die Standzeit eines Alumosilikat-Katalysators ca. 1,5 Jahre. ("Alkylamines", M. Arné, Process Economics Program Report No. 138, 1981, California, USA, Seiten 19 - 41).

Die Standzeit der beschriebenen Alumosilikat-Katalysatoren des Stands der Technik liegt bei maximal 2 Jahren.

Die deutsche Patentanmeldung DE-A-102004062718, (BASF AG) betrifft ein Verfahren zur Herstellung von Methylaminen aus Methanol in Gegenwart eines sauren, formselektiven Molekularsiebs und in Gegenwart von Wasserstoff.

Für die Entwicklung eines Katalysators für das Methylaminverfahren ist es wünschenswert, dass der Katalysator sowohl für die Aminierungs- als auch für die Transalkylierungsreaktion unter den vorgegebenen Reaktionsbedingungen aktiv ist. Mit Aminierungsreaktion ist die Umsetzung von Ammoniak, MMA und/oder DMA mit Methanol und/oder Dimethylether unter Abspaltung von Wasser gemeint. Die Transalkylierung ist die Reaktion von MMA, DMA und/oder TMA mit sich selbst oder mit Ammoniak (siehe: Ullman's Encylopedia of Industrial Chemistry, 5. Auflage, 1996, Vol. A16, Seiten 535 - 541 und Catalysis Today 1997, 37, Seiten 71 - 102).

Beide Reaktionen sind sauer katalysiert, so dass die Azidität des Katalysators, welche durch die Dichte und Stärke der Brönsted- und/oder Lewissauren Zentren bestimmt wird, von entscheidender Bedeutung ist (siehe: Ind. Eng. Chem. Res. 2004, 43, Seiten 5123 - 5132). Die Azidität des Katalysators sollte erfindungsgemäß so optimiert werden, dass eine Aktivierung der Edukte durch Komplexbildung mit dem Aktivzentrum möglich ist, jedoch die Komplexe nicht so stabil sind, dass sich Coke und/oder Cokevorstufen bilden bzw. die Aktivzentren deaktivieren. Eine Möglichkeit die Azidität des Katalysators zu beeinflussen, ist die Einstellung des molaren Al/Si-Verhältnisses.

Ist die Azidität des Katalysators eingestellt, sollte erfindungsgemäß die Verweilzeit der Edukte und Produkte in den Katalysatorformkörpern so optimiert werden, dass eine unter den gegebenen Reaktionsbedingungen ausreichende Diffusion der Edukte und Produkte von und zu den Aktivzentren sowie vom Reaktionsmedium in und aus den Poren des Katalysators möglich ist. Da die Silica-Alumina-Katalysatoren im Allgemeinen nicht mikroporös sind, bedeutet eine Optimierung der Porosität des Katalysators eine Optimierung der Makro- (Porendurchmesser > 25 nm) und/oder Mesoporen (Porendurchmesser 1 - 25 nm).

Zur Verwendung des Katalysators im Produktionsmaßstab sollte die Formkörpergeometrie so gewählt werden, dass der Druckverlust im Reaktor möglichst gering ist. Außerdem sollten die Formkörper unter den gegebenen Reaktionsbedingungen mechanisch so stabil sein, dass Füllhöhen von z.B. bis zu 40 m möglich sind und die Bildung von Abrieb während der Reaktion minimiert wird.

Der vorliegenden Erfindung lag also die Aufgabe zugrunde, einen verbesserten Katalysator und ein verbessertes wirtschaftliches Verfahren zur Herstellung von Methylaminen aufzufinden. Insbesondere soll das Verfahren bei hohem Methanolumsatz (z.B. > 95 %) eine Katalysatorstandzeit von mehr als 2 Jahren gewährleisten.

Demgemäß wurde ein Formkörper enthaltend ein Alumosilikat und Aluminiumoxid gefunden, welcher dadurch gekennzeichnet ist, dass der Formkörper ein molares Al/Si-Verhältnis im Bereich von 10 bis 30 und für Poren mit einem Durchmesser von größer 1 nm eine zumindest bimodale Porenverteilung aufweist, wobei das Volumen der Poren des Formkörpers mit einem Durchmesser von größer 10 nm mindestens 40 % des gesamten Porenvolumes des Formkörpers entspricht.

Die Porenvolumen werden mit Hg-Porosimetrie gemäß DIN 66134 bestimmt.

Weiterhin wurde ein Verfahren zur Herstellung des o.g. Formkörpers gefunden, welches dadurch gekennzeichnet ist, dass es die Schritte
(I) Herstellen eines Gemischs, enthaltend das Alumosilikat, hierunter insbesondere ein Ton, das Aluminiumoxid als Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers
umfasst,
und ein Verfahren zur kontinuierlichen Herstellung von Methylaminen durch Umsetzung von Methanol und/oder Dimethylether mit Ammoniak in Gegenwart eines Heterogen-Katalysators gefunden, welches dadurch gekennzeichnet ist, dass als Katalysator die o.g. Formkörper eingesetzt werden.

Im erfindungsgemäßen Verfahren beträgt die Standzeit des erfindungsgemäßen Katalysators mehr als 3 Jahre, insbesondere mehr als 4 Jahre, und liegt z.B. im Zeitraum von 3,5 bis 6 Jahren.

Zu Herstellung und Eigenschaften der erfindungsgemäßen Formkörper, die im erfindungsgemäßen Verfahren als Katalysator eingesetzt werden:

### Das Alumosilikat (Schritt I)

Als Alumosilikat werden bevorzugt Tone und hierunter insbesondere bevorzugt Schichtsilicate aus der Kaolin-Gruppe eingesetzt (siehe: Ullman's Encylopedia of Industrial Chemistry, 6. Auflage, 2000 elektronische Ausgabe, Kapitel 2 und Lehrbuch der Anorganischen Chemie, A.F. Holleman, E. Wiberg, de Gruyter-Verlag, Berlin, 91.-100. Auflage, 1985, Seiten 771 - 776). Ganz besonders bevorzugt wird Kaolinit {Bruttoformel: Al₂(OH)₄[Si₂O₅]} eingesetzt.

Das Kaolinit ist wesentlicher Bestandteil von Kaolin, welches u. a. in Lagerstätten in Georgien, England, den Vereinigten Staaten, Brasilien, Deutschland, Tschechien, Spanien, Russland und Australien gewonnen wird (siehe: Ullman's Encylopedia of Industrial Chemistry, 6. Auflage, 2000 elektronische Ausgabe, Kapitel 3.1). Das natürliche Kaolin enthält neben Kaolinit geringe Mengen an Feldspaten, Glimmer und Quarz und kann durch dem Fachmann bekannte Verfahren gereinigt werden; hierbei handelt es sich insbesondere um die in Ullman's Encyclopedia of Industrial Chemistry, 6. Auflage, 2000 elektronische Ausgabe, Kapitel 4.1. erwähnten "dry process"- und "wet process"-Verfahren.
Synthetisch kann Kaolinit durch Hydrothermalsynthese aus Polykieselsäure und Aluminiumhydroxid bei pH < 7 erhalten werden.

Der Ton, bevorzugt das Kaolin und besonders bevorzugt das Kaolinit, enthält bevorzugt Spuren an Titan, Eisen, Natrium und Kalium. Der Anteil dieser Elemente liegt bevorzugt für Titan zwischen 0,1 und 1,0 Gew.-%, für Eisen zwischen 0,1 und 1,0 Gew.-%, für Kalium zwischen 0,1 und 5,0 Gew.-% und für Natrium zwischen 0,1 und 5,0 Gew.-%.

Der Anteil an Alumosilikat, insbesondere Ton, bevorzugt Kaolin und insbesondere bevorzugt Kaolinit, im fertigen Katalysatorstrang liegt zwischen 1 und 30 Gew.-%, bevorzugt zwischen 2 und 20 Gew.-% und besonderes bevorzugt zwischen 5 und 15 Gew.-%.

### Das Aluminiumoxid Bindemittel (Schritt I)

Als Bindemittel werden Oxide des Aluminiums und besonderes bevorzugt γ-Al₂O₃ eingesetzt.

Als Vorstufe für das Aluminiumoxid wird in Schritt I Aluminiumhydroxid und/oder Aluminiumoxid/-hydroxid zu dem Gemisch gegeben. Als Aluminiumhydroxid kann sowohl das synthetische Al(OH)₃ oder das naturliche Hydrargillit [γ-Al(OH)₃] eingesetzt werden. Als Aluminiumoxid/-hydroxid [γ-Al(O)OH] werden bevorzugt Böhmit und/oder Pseudoböhmit eingesetzt.

In einer besonderen Ausführungsform wird ein Gemisch aus Aluminiumhydroxid und/oder Aluminiumoxid/-hydroxid und γ-Al₂O₃ als Vorstufe eingesetzt.

In dem Calcinierschritt gemäß V wird insbesondere zumindest 80 % des eingesetzten Aluminiumhydroxids und/oder Aluminiumoxid/-hydroxids in γ-Al₂O₃ umgewandelt.

Der Anteil an Bindemittel im fertigen Formkörper, z.B. Strang, beträgt insbesondere zumindest 50 Gew.-% und liegt bevorzugt zwischen 50 und 99 Gew.-%, besonders bevorzugt zwischen 75 und 95 Gew.-%.

### Das Lösungsmittel (Schritt I)

Als Lösungsmittel, das auch ein Verdünnungsmittel sein kann, wird insbesondere Wasser eingesetzt.

Dem Wasser können sowohl Brönsted-Säuren als auch Brönsted-Basen zugesetzt werden.

Geeignete Brönsted-Säuren sind beispielsweise Salzsäure, Schwefelsäure, Phosphorsäure, Salpetersäure oder Carbonsäuren, Dicarbonsäuren oder Oligo- oder Polycarbonsäuren, wie beispielsweise Nitrilotriessigsäure, Sulfosalicylsäure oder Ethylendiaminotetraessigsäure.

Bevorzugt wird als Brönsted-Säure Ameisensäure oder Salpetersäure verwendet.

Geeignete Brönsted-Basen sind primäre, sekundäre und tertiäre Alkylamine, Ammoniak, sowie Seltenerd-, Alkali- und Erdalkalimetallhydroxide.

Bevorzugt wird als Brönsted-Base Ammoniak verwendet.

Der Anteil an Brönsted-Säuren und/oder Brönsted-Basen im Lösungsmittel (z.B. Wasser) liegt besonders zwischen 0,5 und 99 Gew.-%, bevorzugt zwischen 1 und 50 Gew.-%, besonderes bevorzugt zwischen 5 und 25 Gew.-%.

Die Zugabe des Lösungsmittels bewirkt, dass das Gemisch die richtige Konsistenz für die Weiterverarbeitung im Formungsschritt hat. Der Anteil an Lösungsmittel liegt bevorzugt im Bereich von 0,5 bis 80 Gew.-%, weiter bevorzugt im Bereich von 0,8 bis 50 Gew.-%, weiter bevorzugt im Bereich von 1 bis 40 Gew.-%, und besonders bevorzugt im Bereich von 1,5 bis 30 Gew.-%, jeweils bezogen auf die Gesamtmasse des Gemisches gemäß Schritt I.

In einer besonderen Ausführungsform wird das Gemisch zuerst mit einer Lösung einer Brönsted-Säure (z.B. Ameisensäure) und danach mit einer Lösung einer Brönsted-Base (z.B. NH₃) behandelt.

### Das Anteigmittel (Schritt I)

Bei der Herstellung des Gemisches gemäß (I) wird mindestens ein Anteigmittel (= organischer Zusatzstoff) zugegeben.

Als Anteigmittel (= Anteigungsmittel) können alle dafür geeigneten Verbindungen verwendet werden. Vorzugsweise sind dies organische, insbesondere hydrophile Polymere wie beispielsweise Cellulose, Cellulosederivate, wie beispielsweise Methylcellulose, Stärke, wie beispielsweise Kartoffelstärke, Polyacrylate, Polymethacrylate, Polyvinylalkohol, Polyvinylpyrrolidon (PVP), Polyisobuten (PIB) oder Polytetrahydrofuran (PTHF).

Insbesondere können als Anteigmittel Verbindungen eingesetzt werden, die auch als Porenbildner wirken.

Das Anteigmittel wird in einer besonders bevorzugten Ausführungsform des erfindungsgemäßen Verfahrens, wie untenstehend beschrieben, im Schritt V durch Calcinieren zu mindestens 90 Gew.-% entfernt.

Die Zugabe des Anteigmittels bewirkt, dass das Gemisch die richtige Konsistenz für die Weiterverarbeitung im Formungsschritt hat. Der Anteil an Anteigmittel liegt bevorzugt im Bereich von 0,5 bis 80 Gew.-%, weiter bevorzugt im Bereich von 1 bis 50 Gew.-%, weiter bevorzugt im Bereich von 2 bis 40 Gew.-%, und besonders bevorzugt im Bereich von 3 bis 30 Gew.-%, jeweils bezogen auf die Gesamtmasse des Gemisches gemäß Schritt I.

### Porenbildner (optional, Schritt I)

Das Gemisch aus Bindemittel, Alumosilikat, Anteigmittel und Lösungsmittel gemäß I kann zur weiteren Verarbeitung und zur Ausbildung einer plastischen Masse mit mindestens einer weiteren Verbindung versetzt werden. Unter anderem bevorzugt sind hier Porenbildner zu nennen.

Als Porenbildner können im erfindungsgemäßen Verfahren sämtliche Verbindungen eingesetzt werden, die bezüglich des fertigen Formkörpers eine bestimmte Porengröße, eine bestimmte Porengrößenverteilung und/oder bestimmte Porenvolumina bereitstellen.

Bevorzugt werden als Porenbildner im erfindungsgemäßen Verfahren Polymere eingesetzt, die in Wasser oder in wässrigen Lösungsmittelgemischen dispergier-, suspendier- und/oder emulgierbar sind. Bevorzugte Polymere sind hierbei polymere Vinylverbindungen wie beispielsweise Polyalkylenoxide, wie Polyethylenoxide, Polystyrol, Polyacrylate, Polymethacrylate, Polyolefine, Polyamide und Polyester, Kohlenhydrate, wie Cellulose oder Cellulosederivate, wie beispielsweise Methylcellulose, oder Zucker oder Naturfasern. Weitere geeignete Porenbildner sind Pulp oder Graphit.

Bevorzugt sind auch organische saure Verbindungen, die sich in dem Schritt V, wie untenstehend beschrieben, durch Calcinieren entfernen lassen. Zu nennen sind hier Carbonsäuren, insbesondere C₁₋₈-Carbonsäuren, wie beispielsweise Ameisensäure, Oxalsäure und/oder Zitronensäure. Ebenso ist es möglich, zwei oder mehr dieser sauren Verbindungen einzusetzen.

Werden bei der Herstellung des Gemischs gemäß Schritt I Porenbildner eingesetzt, so liegt der Gehalt des Gemischs gemäß (I) an Porenbildnern bevorzugt im Bereich von 0,5 bis 80 Gew.-%, weiter bevorzugt im Bereich von 1 bis 50 Gew.-% und besonders bevorzugt im Bereich von 2 bis 30 Gew.-%, jeweils bezogen auf die Gesamtmasse im Gemisch gemäß (I).

Sollte dies für die zu erzielende Porengrößenverteilung erwünscht sein, kann auch ein Gemisch aus zwei oder mehr Porenbildnern eingesetzt werden.

Die Porenbildner werden in einer besonders bevorzugten Ausführungsform des erfindungsgemäßen Verfahrens, wie untenstehend beschrieben, im Schritt V durch Calcinieren unter Erhalt des porösen Formkörpers zu mindestens 90 Gew.-% entfernt.

### Vermischen und Verdichten (Schritt II)

Nach der Herstellung des Gemisches gemäß (I) wird das Gemisch homogenisiert, z.B. für eine Dauer im Bereich von 10 bis 180 Minuten. Besonders bevorzugt werden zur Homogenisierung unter anderem Kneter, Koller oder Extruder eingesetzt. Im kleineren Maßstab wird das Gemisch bevorzugt geknetet. Im industriellen, größeren Maßstab wird zur Homogenisierung bevorzugt gekollert.

Bei der Homogenisierung wird bevorzugt bei Temperaturen im Bereich von ungefähr 10°C bis zum Siedepunkt des Lösungsmittels und Normaldruck oder leichtem überatmosphärischem Druck gearbeitet. Danach kann gegebenenfalls mindestens eine der oben als Anteigmittel oder Porenbildner beschriebenen Verbindungen zugegeben werden. Das so erhaltene Gemisch wird solange homogenisiert, vorzugsweise geknetet, bis eine verstrangbare plastische Masse entstanden ist.

Das homogenisierte Gemisch wird in einem folgenden Schritt verformt.

### Verformen des verdichteten Gemischs unter Erhalt eines Formkörpers (Schritt III)

Zur Durchführung dieses Schritts sind solche Verfahren bevorzugt, bei denen die Verformung durch Extrusion in üblichen Extrudern, beispielsweise zu Strängen mit einem Durchmesser von bevorzugt 1 bis 20 mm und besonders bevorzugt 2 bis 15 mm, z.B. 1 bis 10 oder 2 bis 5 mm, erfolgt. Derartige Extrusionsvorrichtungen werden beispielsweise in Ullmann's Enzyklopädie der Technischen Chemie, 4. Auflage, Bd. 2, S. 295 ff., 1972, beschrieben.
Neben der Verwendung eines Extruders wird ebenfalls vorzugsweise eine Strangpresse zur Verformung verwendet.

Prinzipiell können jedoch zur Formgebung alle bekannten und/oder geeigneten Knet- und Verformungsvorrichtungen bzw. Verfahren eingesetzt werden. Unter anderem sind hierbei zu nennen:
(i) Brikettieren, d.h. mechanisches Verpressen mit oder ohne Zusatz von zusätzlichem Bindermaterial;
(ii) Pelletieren, d.h. Kompaktieren durch kreisförmige und/oder rotierende Bewegungen;
(iii) Sintern, d.h. das zu verformende Materials wird einer thermischen Behandlung ausgesetzt.

Beispielsweise kann die Formgebung aus der folgenden Gruppe ausgewählt sein, wobei die Kombination von mindestens zwei dieser Methoden explizit eingeschlossen ist: Brikettieren durch Stempelpressen, Walzenpressen, Ringwalzenpressen, Brikettieren ohne Bindemittel; Pelletieren, Schmelzen, Spinning-Techniken, Abscheidung, Schäumen, Sprühtrocknen; Brennen im Schachtofen, Konvektionsofen, Wanderrost, Drehrohrofen, Kollern.

Das Kompaktieren kann bei Umgebungsdruck oder bei gegenüber dem Umgebungsdruck erhöhtem Druck stattfinden, beispielsweise in einem Druckbereich von 1 bar bis zu mehreren hundert bar. Weiterhin kann das Kompaktieren bei Umgebungstemperatur oder bei gegenüber der Umgebungstemperatur erhöhter Temperatur stattfinden, beispielsweise in einem Temperaturbereich von 20 bis 300°C. Ist Trocknen und/oder Brennen Bestandteil des Formgebungsschritts, so sind Temperaturen bis zu 1500 °C anwendbar. Schließlich kann das Kompaktieren in der UmgebungsAtmosphäre stattfinden oder in einer kontrollierten Atmosphäre. Kontrollierte Atmosphären sind beispielsweise Schutzgas-Atmosphären, reduzierende oder oxidierende Atmosphären.

Die Form der erfindungsgemäß hergestellten Formkörper kann beliebig gewählt werden. Insbesondere sind unter anderem Kugeln, ovale Formen, Zylinder, zylinderförmige Stränge oder Tabletten möglich.

Bei den Extrudate beträgt das Länge : Durchmesser - Verhältnis insbesondere mindestens 1, liegt bevorzugt im Bereich von größer 1 bis 20, besonders bevorzugt im Bereich von 2 bis 10.

### Trocknen des Formkörpers (Schritt IV)

Dem Schritt (III) schließt sich im Rahmen der vorliegenden Erfindung bevorzugt mindestens ein Trocknungsschritt an. Dieser mindestens eine Trocknungsschritt erfolgt dabei bei Temperaturen im Bereich von bevorzugt 80 bis 160°C, insbesondere von 90 bis 145°C und besonders bevorzugt von 100 bis 130°C, wobei die Trocknungsdauer bevorzugt 6 h oder mehr, beispielsweise im Bereich von 6 bis 24 h, beträgt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Vor und/oder nach dem Trocknungsschritt kann das bevorzugt erhaltene Extrudat beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

### Calcinierung des Formkörpers (Schritt V)

Dem Schritt (IV) folgt mindestens ein Calcinierungsschritt. Die Calcinierung wird bei Temperaturen im Bereich von bevorzugt 350 bis 750°C und insbesondere von 450 bis 700°C durchgeführt.

Die Calcinierung kann unter jeder geeigneten Gasatmosphäre erfolgen, wobei Luft und/oder Magerluft bevorzugt sind.
Das Calcinieren gemäß (V) kann auch in Gegenwart von Wasserstoff, Stickstoff, Helium, Argon und/oder Dampf oder Gemischen hiervon erfolgen.

Weiter wird die Calcinierung bevorzugt in einem Muffelofen, einem Drehrohrofen oder einem Bandkalzinierofen durchgeführt, wobei die Calcinierungsdauer bevorzugt bei 1 h oder mehr, beispielsweise im Bereich von 1 bis 24 h oder im Bereich von 3 bis 12 h liegt. Demgemäß ist es im Rahmen des erfindungsgemäßen Verfahrens beispielsweise möglich, den Formkörper einmal, zweimal oder öfter für jeweils mindestens 1 h, wie beispielsweise jeweils im Bereich von 3 bis 12 h, zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Nach dem Calcinierungsschritt kann das calcinierte Material beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

Die erhaltenen Formkörper weisen Härten auf, die bevorzugt im Bereich von 2 bis 200 N (Newton), besonders bevorzugt im Bereich von 5 bis 150 N und ganz besonders bevorzugt mindestens 10 N betragen, z.B. im Bereich von ≥ 10 bis 100 N liegen.

Die obenstehend beschriebene Härte wurde im Rahmen der vorliegenden Erfindung an einem Apparat der Firma Zwick, Typ BZ2.5/TS1 S mit einer Vorkraft von 0,5 N, einer Vorkraftschubgeschwindigkeit von 10 mm/Min. und einer nachfolgenden Prüfgeschwindigkeit von 1,6 mm/Min. bestimmt. Das Gerät besaß einen festsitzenden Drehteller und einen frei beweglichen Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller hin, auf der der zu untersuchende Katalysatorformkörper lag. Das Prüfgerät wurde über einen Computer gesteuert, der die Messergebnisse registrierte und auswertete. Die erzielten Werte stellen den Mittelwert aus den Messungen zu jeweils mindestens 10 Katalysatorformkörpern dar.

Die erhaltenen Formkörper weisen bevorzugt ein molares Al/Si-Verhältnis im Bereich von 10 bis 35, insbesondere im Bereich von 11 bis 26, auf. Z.B. liegt das molare Al/Si-Verhältnis im Bereich von 12 bis 25, besonders 14 bis 23.
Die Angaben zum molaren Al/Si-Verhältnis im Formkörper beziehen sich auf den Gesamtgehalt an Al und Si.

Die Azidität der Formkörper wird durch das molare Al/Si-Verhältnis im Formkörper vorgegeben. Im Bereich des molaren Al/Si-Verhältnisses von 10 bis 30 steigt die Azidität bei einer Abnahme des molaren Al/Si-Verhältnisses.

Die Azidität wird bestimmt mit Hilfe von FT-IR-Spektroskopie durch Messung der Intensität der Sorptionsbanden bei v = 1490 ± 2 cm⁻¹ und/oder 1440 ± 2 cm⁻¹ von mit Pyridin begasten Proben (Presslingen) bei 80°C und 1,0 mbar analog der Methode beschrieben in Journal of Catalysis 1963, 2, Seiten 371 - 379. Die Intensität der Banden wird als Extinktion bezogen auf die Schichtdicke der gemessenen Presslinge als integrale Extinktionseinheit (IEE) pro µm Schichtdicke angegeben.

Für die Sorptionsbande bei v = 1490 ± 2 cm⁻¹ liegt der Wert der IEE/µm - Einheit bei einem Pyridin-Partialdruck von 1,0 mbar bevorzugt im Bereich zwischen 0,001 und 0,015 und besonderes bevorzugt im Bereich zwischen 0,003 und 0,010.
Für die Sorptionsbande bei v = 1440 ± 2 cm⁻¹ liegt der Wert der IEE/µm - Einheit bei einem Pyridin-Partialdruck von 1,0 mbar bevorzugt im Bereich zwischen 0,020 und 0,100 und besonderes bevorzugt im Bereich zwischen 0,025 und 0,060.

Die Sorptionsbanden im FT-IR-Spektrum bei v = 1490 ± 2 cm-¹ und 1440 ± 2 cm-¹ der mit Pyridin begasten Proben entsprechen der Koordination von Pyridin mit Lewissauren Zentren (siehe Journal of Catalysis 1963, 2, Seiten 371 - 379, Catalysis and Zeolites (Eds.: J. Weitkamp, L. Puppe), 1999, Springer Verlag, Berlin, Kapitel 4.1.5.1.3, Seiten 224 - 226 und Catalysis Today 2001, 68, Seiten 263 - 381). Die für Brönstedsaure Zentren im IR-Spektrum typische Sorptionsbande bei v = 1540 ± 2 cm⁻¹ einer mit Pyridin begasten Probe wird für die erfindungsgemäßen und erfindungsgemäß eingesetzten Alumosilikat-Formkörper nicht beobachtet.

Die spezifische Oberfläche der erhaltenen Formkörper, bestimmt gemäß DIN 66131 (BET), liegt bevorzugt bei mindestens 50 m²/g und insbesondere bevorzugt bei mindestens 100 m²/g. Beispielsweise liegt die spezifische Oberfläche im Bereich von 50 bis 250 m²/g und insbesondere im Bereich von 100 bis 200 m²/g.

Das Porenvolumen der erhaltenen Formkörper, bestimmt gemäß DIN 66134 (Hg-Porosimetrie), liegt bevorzugt bei mindestens 0,4 ml/g, besonders bevorzugt bei mindestens 0,6 ml/g. Beispielsweise liegt das Porenvolumen im Bereich von 0,4 bis 1,5 ml/g und insbesondere im Bereich von 0,6 bis 1,0 ml/g.

Die Poren der erhaltenen Formkörper mit einem Durchmesser > 1 nm, bestimmt gemäß DIN 66134 (Hg-Porosimetrie), weisen eine multimodale, zumindest bimodale Verteilung, bevorzugt eine bimodalen Verteilung auf, wobei der Anteil an Poren mit einem Durchmesser > 10 nm minimal 40 %, bevorzugt minimal 50 %, des Gesamtporenvolumens einnimmt und z.B. im Bereich von 40 bis 60 % liegt.

Die Formkörper enthalten bevorzugt Spuren an Titan, Eisen, Natrium und Kalium. Der Anteil dieser Elemente liegt bevorzugt für Titan zwischen 0,01 und 0,35 Gew.-%, für Eisen zwischen 0,01 und 0,35 Gew.-%, für Kalium zwischen 0,01 und 1,75 Gew.-% und für Natrium zwischen 0,01 und 1,75 Gew.-%, jeweils bezogen auf das Formkörpergewicht.

### Herstellung von Methylaminen an dem erfindungsgemäßen Katalysator:

Die Herstellung von Methylaminen an dem erfindungsgemäßen Katalysator erfolgt durch Umsetzung von Ammoniak und Methanol und/oder Dimethylether in der Gasphase bei erhöhtem Druck und erhöhter Temperatur. Wahlweise kann dem/der Zulauf für die Reaktion auch Wasser und/oder Monomethylamin und/oder Dimethylamin und/oder Trimethylamin zugesetzt werden bzw. enthalten.

Die Katalysatorbelastung wird als Masse an kohlenstoffhaltigen Verbindungen im Zulauf, gerechnet als Methanol, bezogen auf das Katalysatorvolumen (L = Liter) und die Zeit ausgedrückt. Die Katalysatorbelastung liegt bevorzugt im Bereich von 0,1 bis 2,0 kg(Methanol)/L(Katalysator)/h, insbesondere im Bereich von 0,2 bis 1,5 kg/Uh, ganz besonders im Bereich von 0,4 bis 1,0 kg/L/h.

Das molare N/C-Verhältnis bezogen auf die Summe der Einsatzstoffe liegt bevorzugt im Bereich von 0,6 bis 4,0, insbesondere 0,8 bis 2,5, ganz besonders 1,0 bis 2,0.

Die Umsetzung wird bevorzugt bei einer Temperatur im Bereich von 250 bis 500°C, besonders 300 bis 475°C, ganz besonders 350 bis 450°C, durchgeführt.

Der Absolutdruck der Umsetzung liegt bevorzugt im Bereich von 5 bis 50 bar, besonders 10 bis 30 bar, insbesondere 15 bis 25 bar.

Der Umsatz (= U) an Methanol liegt bevorzugt bei > 85 %, besonderes bevorzugt zwischen 90 % und 99 %, insbesondere zwischen 95 % und 99 %.

Die Selektivität (= S) der Umsetzung bezüglich Mono-, Di- und Trimethylamin liegt bevorzugt bei > 90 %, besonderes bevorzugt bei > 95 %.

Die im erfindungsgemäßen Verfahren eingesetzten Formkörper bilden bevorzugt ein Katalysatorfestbett, z.B. hergestellt durch Schüttung.
Die Umsetzung wird bevorzugt in einem adiabat betriebenen Schachtreaktor durchgeführt.

In einer besonderen Ausgestaltung des erfindungsgemäßen Verfahrens zur Herstellung von Methylaminen werden als Katalysator die erfindungsgemäßen Formkörper eingesetzt, wobei diese ein Festbett bilden, das zwei, drei, vier oder mehr, bevorzugt zwei, verschiedene Formkörpertypen enthält, die sich bezüglich ihrer Azidität unterscheiden.

Die Anordnung der zumindest zwei unterschiedlichen Formkörpertypen (Formkörpertyp A, Formkörpertyp B, ...) kann in Form eines homogenen Gemisches oder in Form von Schichten, die jeweils aus den verschiedenen Formkörpertypen aufgebaut sind, erfolgen. Bei dem Einbau von zwei Schichten werden die Formkörpertypen (A) mit kleinerem Al/Si-Verhältnis bevorzugt in Strömungsrichtung nach den Formkörpertypen (B) mit größerem Al/Si-Verhältnis eingebaut.

Im Fall von zwei verschiedenen Formkörpertypen im Katalysatorfestbett liegt das Verhältnis der Formkörpertypen A und B (A : B) im Bett zwischen 10 : 90 Gew.-% und 90 : 10 Gew.-%, bevorzugt zwischen 20 : 80 Gew.-% und 80 : 20 Gew.-% und besonders bevorzugt zwischen 30 : 70 Gew.-% und 70 : 30 Gew.-%.

Der Unterschied des molaren Al/Si-Verhältnisses der Formkörpertypen A und B beträgt maximal 20, liegt bevorzugt im Bereich von ≥ 2 bis ≤ 20, besonders bevorzugt im Bereich von ≥ 3 bis ≤ 10.

Die Aufarbeitung des Reaktoraustrags kann in Anlehnung an dem Fachmann bekannten Verfahren, z.B. gemäß DD-125 533 (VEB Leuna-Werke), erfolgen.

Optional kann das Verfahren zur Herstellung von Methylaminen mit dem erfindungsgemäßen Katalysator in Kombination mit einer Verschaltung mit einem Reaktor enthaltend einen formselektiven Katalysator gemäß US 4,485,261 und/oder PEP-Review, No. 89-3-4, (SRI International, Menlo Park, California) durchgeführt werden.

Bei dem jeweils gegebenenfalls eingesetzten Dimethylether (DME), Trimethylamin (TMA) und/oder Monomethylamin (MMA) handelt es sich in besonderen Ausgestaltungen der Erfindung um einen jeweiligen Rückführstrom aus dem aufgearbeiteten Reaktionsprodukt des Verfahrens.

### Regenerierung der Katalysatorformkörper nach ihrer Verwendung in der kontinuierlichen Synthese von Methylaminen:

In einer weiteren Ausführungsform des erfindungsgemäßen Verfahrens wird der Katalysator nach dem Einsatz unabhängig von seiner Form, z.B. nach Abnahme der Aktivität und/oder der Selektivität, durch ein Verfahren regeneriert, bei dem die Regenerierung durch gezieltes Abbrennen (bei z.B. einer Temperatur im Bereich von 350 bis 650°C) der für die Deaktivierung verantwortlichen Beläge erfolgt. Dabei wird bevorzugt in einer Inertgasatmosphäre gearbeitet, die genau definierte Mengen an Sauerstoff oder Sauerstoff liefernden Substanzen enthält. Ein solches Regenerierungsverfahren ist unter anderem in der WO-A-98/55228 und der DE-A1-197 23 949 und insbesondere für Katalysatoren zur Herstellung von Methylaminen in der JP-08 157 428 und der EP-A-0118 193 beschrieben, deren diesbezügliche Offenbarung durch Bezugnahme hiermit vollumfänglich in den Gegenstand der vorliegenden Anmeldung einbezogen wird.

Nach der Regeneration sind die Aktivität und/oder die Selektivität des Katalysators, verglichen mit dem Zustand unmittelbar vor der Regeneration, erhöht.

Der zu regenerierende, erfindungsgemäße Katalysator wird entweder in der Umsetzungsvorrichtung (Reaktor) oder in einem externen Ofen in einer Atmosphäre, die bevorzugt 0,1 bis ungefähr 20 Volumen-Anteile von Sauerstoff liefernden Substanzen, besonders bevorzugt 0,1 bis 20 Volumenanteile Sauerstoff, enthält, auf eine Temperatur im Bereich von 350°C bis 800°C, vorzugsweise von 400°C bis 650°C und insbesondere von 425°C bis 500°C aufgeheizt. Dabei wird das Aufheizen vorzugsweise mit einer Aufheizrate von 0,1 °C/Min. bis 20°C/Min., vorzugsweise von 0,3°C/Min. bis 15°C/Min. und insbesondere von 0,5°C/Min. bis 10°C/Min. durchgeführt. Das Aufheizen wird bevorzugt in einer Inertatmosphäre durchgeführt.

Während der Regenerierung wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur oder des Reaktors kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators. Die Gasbelastung des Sauerstoff enthaltenden Regeneriergases ausgedrückt als GHSV (= gas hourly space velocity) liegt bei mehr als 50 Normliter pro Liter Katalysator und Stunde (= NL/L(Katalysator)/h), bevorzugt bei mehr als 100 NL/L/h und besonderes bevorzugt im Bereich zwischen 150 und 1000 NL/L/h.
(NL = Normliter = auf Normalbedingungen umgerechnetes Volumen).

Sinkt die Temperatur des Abgasstroms am Reaktorausgang auf die Temperatur am Reaktoreingang trotz steigender Mengen an Sauerstoff oder Sauerstoff liefernden Substanzen im Gasstrom und/oder steigt die Konzentration an Sauerstoff im Reaktionsaustrag auf den Eingangswert an, so ist das Abbrennen der organischen Beläge beendet. Die Dauer der Behandlung beträgt bevorzugt jeweils 1 bis 72 h, vorzugsweise ungefähr 2 bis ungefähr 48 h und insbesondere ungefähr 3 bis ungefähr 24 Stunden.

Das anschließende Abkühlen des so regenerierten Katalysators wird bevorzugt so durchgeführt, dass das Abkühlen nicht zu schnell erfolgt, da sonst die mechanische Festigkeit des Katalysators negativ beeinflusst werden kann. Das Abkühlen wird bevorzugt in einer Inertatmosphäre durchgeführt.

Es kann erforderlich sein, den Katalysator nach der durchgeführten Regeneration durch Calcinieren, wie oben beschrieben, einer Spülung mit Wasser und/oder verdünnten Säuren wie beispielsweise Salzsäure zu unterziehen, um die durch Verunreinigung der Edukte gegebenenfalls verbleibende anorganische Beladung des Katalysators (Alkalispuren etc.) zu entfernen. Anschließend kann eine erneute Trocknung und/oder ein erneutes Calcinieren des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im Allgemeinen getrocknet. Obwohl der Trocknungsvorgang im Allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren zu vermeiden, da dies zu Schädigungen des Katalysators führen kann.

Eine bevorzugte Ausführung des Herstellverfahrens besteht darin, dass das erfindungsgemäße, kontinuierliche Verfahren zur Synthese von Methylaminen bei der Regeneration des erfindungsgemäßen Katalysators nicht unterbrochen werden muss, um so den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung des Eduktes oder der Edukte zur Verfügung steht.

### Beispiele

Die BET-Oberflächen (m²/g) und die Porenvolumina (ml/g) wurden nach DIN 66131 bzw. DIN 66134 Norm bestimmt.

### GC-Analytik:

Die Reaktionsausträge wurden mittels Online-Gaschromatographie analysiert. Die Trennung der Methylamine erfolgte dabei auf einer für kurzkettige Amine optimierten GC-Säule (Varian CP-Volamine), zur Detektion wurde ein Wärmeleitfähigkeitsdetektor (WLD) eingesetzt. Der Gehalt an nicht umgesetzten Methanol wurde bestimmt, woraus auf die Aktivität des Katalysators geschlossen wurde.

Die Bestimmung/Messung der Schneidhärte erfolgte wie in der WO-A-04/108280 (BASF AG) beschrieben (siehe auch weiter oben in der Beschreibung).

### Beispiel: Herstellung des Katalysators A (erfindungsgemäß)

177 g gamma-Al₂O₃ wurde zusammen mit 222 g Böhmit und 44 g Kaolin mechanisch gemischt und anschließend mit 200 g 4,2%-iger Ameisensäure versetzt. Danach wurde die Masse mechanisch gemischt, mit 90 g wässriger Stärkelösung und 9 g 25%-iger wässriger NH₃-Lösung versetzt. Die so erhaltene Masse wurde in einem Extruder zu 4 mm Strängen verformt. Die Stränge wurden in einem Bandtrockner 2 h bei 120°C getrocknet und anschließend in einem Muffelofen 2 h bei 610 °C kalziniert.

Der erhaltene Katalysator weist ein molares Al/Si-Verhältnis von 17,3 auf. Der Anteil an Eisen, Kalium, Natrium und Titan liegt bei 0,03, 0,21, 0,01 bzw. 0,06 Gew.-%. Der Formkörper hat eine bimodale Porenverteilung, die Porendurchmesser betragen 7,3 und 11,0 nm. Das Gesamtporenvolumen der Poren mit Durchmesser > 1 nm beträgt 0,49 ml/g. Der Volumenanteil der Poren mit einem Durchmesser > 10 nm liegt bei 56 %.

Die Schneidhärte beträgt 68,5 N und die BET-Oberfläche 206 m²/g.

Beispiele: Kontinuierliche Herstellung von Methylaminen durch Umsetzung von Methanol mit Ammoniak in Gegenwart des erfindungsgemäßen Katalysators

### A)

In einer technischen Anlage zur Herstellung von Methylaminen wurden erfindungsgemäße Katalysatoren für die Methylamin-Synthese aus Methanol, Ammoniak sowie Dimethylether, Monomethylamin und Trimethylamin eingesetzt. Nach einer Laufzeit von 5 Jahren wurde ein Katalysatorwechsel durchgeführt. Der Kohlenstoffgehalt des ausgebauten Katalysators betrug 1,7 Gew.-%. Die Anlage wurde nachfolgend 4 Jahre ohne Katalysatorwechsel betrieben. Der nach 4 Jahren ausgebaute Katalysator wies einen Kohlenstoffgehalt von 1,3 Gew.-% auf. Die Katalysatoren besaßen zum Zeitpunkt des Ausbaus jeweils eine gute Aktivität (Methanolumsätze > 95 %), der Katalysatorwechsel erfolgte, weil technische Umbauten die Abstellung der Anlage erforderten.

### B)

Ein erfindungsgemäßer Katalysator wurde als 2 mm Stränglinge und als 4 mm Stränge eingesetzt. Die Methylamin-Synthese erfolgte im geraden Durchgang ohne Rückführung von Reaktionskomponenten. Die Versuche wurden bei Reaktionstemperaturen von 350 bis 430 °C und Belastungen von 0,4 bis 1,2 kg (MeOH)/Liter Katalysator/h durchgeführt. Das molare N/C-Verhältnis wurde zwischen 1,2:1 und 1,8:1 variiert.
Die Versuche wurden in einem 1 Liter Rohrreaktor einem Durchmesser von 30 mm durchgeführt. Der Reaktor wurde isotherm gefahren (elektrische Heizung mit drei Heizkreisen).

### GC-Analytik

Trennsäule Varian CP-Volamine (WCOT Fused Silica), Länge 60 m, Innendurchmesser: 0,32 mm; Trägergas Helium; Temperaturprogramm: 10 Minuten isotherm bei 40 °C, danach mit 20 °C/Minuten auf 250 °C, abschließend 5 Minuten isotherm bei 250 °C.

Zur Katalysatorevaluation wurde eine Vielzahl von Versuchen bei unterschiedlichen Temperaturen und Lasten durchgeführt. Bei Verwendung der 4 mm Stränge des Katalysators ergab sich in der Laufzeit von 1300 h keine Druckdifferenz im Reaktor. Die Katalysatoraktivität war weiterhin sehr gut. Der Kohlenstoffgehalt des ausgebauten Katalysators betrug zwischen 0,4 und 0,6 Gew.%.

### Versuchsergebnisse

**Tabelle 1: Versuchsergebnisse zur Methylamin-Synthese bei Molverhältnis 1,2:1**

| Belastung Molverhältnis | Temperatur | Laufzeit absolut in h | Durchmesser | U (MeOH) | MMA | DMA | TMA |
|---|---|---|---|---|---|---|---|
| | | | Stränge | | Gew.% | Gew.% | Gew.% |
| 0,8 kg (MeOH)/(L*h) | 370 °C | 1065-1100 | 2 mm | 95,3 | 17,5 | 19,6 | 62,9 |
| N/C=1,2:1 mol/mol | | 339-383 | 4 mm | 86,7 | 20,2 | 17,0 | 62,8 |
| | 380 °C | 935-983 | 4 mm | 91,5 | 17,7 | 17,2 | 65,1 |
| | 390°C | 479-527 | 4 mm | 94,8 | 17,3 | 18,6 | 64,0 |
| | 400 °C | 527-575 | 4 mm | 98,3 | 18,2 | 22,7 | 59,2 |
| | 410°C | 695-743 | 4 mm | 98,7 | 19,6 | 24,7 | 55,8 |
| | 420 °C | 743-791 | 4 mm | 98,8 | 21,0 | 26,4 | 52,6 |
| | 430 °C | 887-935 | 4 mm | 98,7 | 22,4 | 27,1 | 50,6 |
| 1,2 kg (MeOH)/(L*h) | 380 °C | 1029-1065 | 2 mm | 94,7 | 17,5 | 19,1 | 63,4 |
| N/C=1,2:1 mol/mol | | 383-431 | 4 mm | 86,3 | 19,7 | 16,9 | 63,4 |
| | WDH | 983-1031 | 4 mm | 86,4 | 18,8 | 16,6 | 64,6 |
| | 390 °C | 431-479 | 4 mm | 89,2 | 18,8 | 17,4 | 63,9 |
| | 400 °C | 575-623 | 4 mm | 92,8 | 17,7 | 18,1 | 64,2 |
| | 410 °C | 623-695 | 4 mm | 95,8 | 17,3 | 19,9 | 62,8 |
| | 420 °C | 791-839 | 4 mm | 98,3 | 18,9 | 23,9 | 57,2 |
| | 430 °C | 839-887 | 4 mm | 98,6 | 20,3 | 25,6 | 54,0 |

**Tabelle 2: Versuchsergebnisse zur Methylamin-Synthese bei Molverhältnis 1,8:1**

| Belastung Molverhältnis | Temperatur | Laufzeit absolut in h | Durchmesser Stränge | U (MeOH) | MMA | DMA | TMA |
|---|---|---|---|---|---|---|---|
| | | | | | Gew.% | Gew.% | Gew.% |
| 0,4 kg (MeOH)/(L*h) | 350 °C | 935-968 | 2 mm | 90,7 | 24,2 | 18,9 | 56,9 |
| N/C=1,8:1 mol/mol | | 1247-1295 | 4 mm | 88,9 | 24,9 | 18,2 | 56,9 |
| | | | | | | | |
| | 360 °C | 793-837 | 2 mm | 95,7 | 23,7 | 21,3 | 55,0 |
| | | 1295-1343 | 4 mm | 93,5 | 23,5 | 19,2 | 57,3 |
| 0,8 kg (MeOH)/(L*h) | 360 °C | 758-793 | 2 mm | 86,4 | 25,5 | 18,4 | 56,2 |
| N/C=1,8:1 mol/mol | | 0-181 | 4 mm | 81,6 | 27,8 | 18,8 | 53,4 |
| | WDH | 1199-1247 | 4 mm | 82,4 | 26,2 | 18,0 | 55,8 |
| | | | | | | | |
| | 370 °C | 637-757 | 2 mm | 92,7 | 23,7 | 19,7 | 56,6 |
| | | 1151-1199 | 4 mm | 88,0 | 24,5 | 18,4 | 57,1 |
| | | | | | | | |
| | 380 °C | 590-638 | 2 mm | 96,8 | 24,4 | 22,6 | 53,0 |
| | | 181-239 | 4 mm | 90,9 | 24,4 | 21,5 | 54,1 |
| 1,2 kg (MeOH)/(L*h) | 380 °C | 968-993 | 2 mm | 89,8 | 25,0 | 19,5 | 55,5 |
| N/C=1,8:1 mol/mol | | 239-287 | 4 mm | 85,0 | 26,7 | 19,0 | 54,3 |
| | | | | | | | |
| | 385 °C | 993-1029 | 2 mm | 93,2 | 24,5 | 20,7 | 54,7 |
| | | 287-335 | 4 mm | 86,9 | 20,8 | 20,6 | 58,6 |
| WDH = Wiederholung | | | | | | | |

## Patentansprüche

1. Formkörper enthaltend ein Alumosilikat und Aluminiumoxid, **dadurch gekennzeichnet, dass** der Formkörper ein molares Al/Si-Verhältnis im Bereich von 10 bis 35 und für Poren mit einem Durchmesser von größer 1 nm eine zumindest bimodale Porenverteilung aufweist, wobei das Volumen der Poren des Formkörpers mit einem Durchmesser von größer 10 nm mindestens 40 % des gesamten Porenvolumes des Formkörpers entspricht.

2. Formkörper nach Anspruch 1, **dadurch gekennzeichnet, dass** er ein molares Al/Si-Verhältnis im Bereich von 11 bis 26 aufweist.

3. Formkörper nach Anspruch 1 oder 2, **dadurch gekennzeichnet, dass** er für Poren mit einem Durchmesser von größer 1 nm eine bimodale Porenverteilung aufweist.

4. Formkörper nach einem der vorhergehenden Ansprüche, **dadurch gekennzeichnet, dass** das Volumen der Poren des Formkörpers mit einem Durchmesser von größer 10 nm mindestens 50 % des gesamten Porenvolumes des Formkörpers entspricht.

5. Formkörper nach einem der vorhergehenden Ansprüche, **dadurch gekennzeichnet, dass** er eine BET-Oberfläche von ≥ 50 m²/g aufweist.

6. Formkörper nach einem der Ansprüche 1 bis 4, **dadurch gekennzeichnet, dass** er eine BET-Oberfläche im Bereich von 100 bis 250 m²/g aufweist.

7. Formkörper nach einem der vorhergehenden Ansprüche, **dadurch gekennzeichnet, dass** der Formkörper im Bereich von 0,01 bis 1,75 Gew.-% Natrium, 0,01 bis 1,75 Gew.-% Kalium, 0,01 bis 0,35 Gew.-% Titan und 0,01 bis 0,35 Gew.-% Eisen, jeweils bezogen auf das Gesamtgewicht, enthält.

8. Formkörper nach einem der vorhergehenden Ansprüche, **dadurch gekennzeichnet, dass** es sich um ein Extrudat mit einem Länge : Duchmesser - Verhältnis von ≥ 1 handelt.

9. Formkörper nach einem der vorhergehenden Ansprüche, **dadurch gekennzeichnet, dass** er eine Schneidhärte von ≥ 10 Newton (N) aufweist.

10. Formkörper nach einem der vorhergehenden Ansprüche, **dadurch gekennzeichnet, dass** es sich bei dem Alumosilikat um ein Ton handelt.

11. Formkörper nach einem der vorhergehenden Ansprüche, **dadurch gekennzeichnet, dass** es sich bei dem Alumosilikat um ein Kaolin handelt.

12. Formkörper nach einem der vorhergehenden Ansprüche, **dadurch gekennzeichnet, dass** es sich bei dem Alumosilikat um Kaolinit handelt.

13. Formkörper nach einem der vorhergehenden Ansprüche, **dadurch gekennzeichnet, dass** es sich bei dem Aluminiumoxid um gamma-Al₂O₃ handelt.

14. Formkörper nach dem vorhergehenden Anspruch, **dadurch gekennzeichnet, dass** als Vorstufe für das gamma-Aluminiumoxid Aluminiumhydroxid und/oder Aluminiumoxid/-hydroxid (Böhmit) verwendet wird.

15. Formkörper nach einem der vorhergehenden Ansprüche, **dadurch gekennzeichnet, dass** der Anteil an Aluminiumoxid im Formkörper ≥ 50 Gew.-% beträgt.

16. Verfahren zur Herstellung eines Formkörpers gemäß einem der vorhergehenden Ansprüche, **dadurch gekennzeichnet, dass** es die Schritte
(I) Herstellen eines Gemischs, enthaltend das Alumosilikat, das Aluminiumoxid als Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers
umfasst.

17. Verfahren nach Anspruch 16, **dadurch gekennzeichnet, dass** in Schritt (I) das Gemisch einen Porenbildner enthält.

18. Verfahren nach Anspruch 16 oder 17, **dadurch gekennzeichnet, dass** in Schritt (I) der Anteil an Alumosilikat so ausgewählt wird, dass er im fertigen Formkörper zwischen 1 und 30 Gew.-% beträgt.

19. Verfahren nach einem der Ansprüche 16 bis 18, **dadurch gekennzeichnet, dass** in Schritt (I) der Anteil an Aluminiumoxid so ausgewählt wird, dass er im fertigen Formkörper zumindest 50 Gew.-% beträgt.

20. Verfahren nach einem der Ansprüche 16 bis 19, **dadurch gekennzeichnet, dass** das Verformen gemäß (III) durch Extrusion erfolgt.

21. Verfahren nach einem der Ansprüche 16 bis 20, **dadurch gekennzeichnet, dass** das Calcinieren gemäß (V) bei einer Temperatur im Bereich von 350 bis 750°C und einer Dauer im Bereich von 1 bis 24 h erfolgt.

22. Verfahren zur kontinuierlichen Herstellung von Methylaminen durch Umsetzung von Methanol und/oder Dimethylether mit Ammoniak in Gegenwart eines Heterogen-Katalysators, **dadurch gekennzeichnet, dass** als Katalysator Formkörper gemäß einem der Ansprüche 1 bis 15 eingesetzt werden.

23. Verfahren nach dem vorhergehenden Anspruch, **dadurch gekennzeichnet, dass** der Feedstrom neben Methanol und/oder Dimethylether und Ammoniak Monomethylamin und/oder Dimethylamin und/oder Trimethylamin enthält.

24. Verfahren nach einem der beiden vorhergehenden Ansprüche, **dadurch gekennzeichnet, dass** das molare N/C-Verhältnis im Feedgemisch im Bereich von 0,6 bis 4,0 liegt.

25. Verfahren nach einem der drei vorhergehenden Ansprüche, **dadurch gekennzeichnet, dass** die Reaktionstemperatur im Bereich von 250 bis 500°C liegt.

26. Verfahren nach einem der vier vorhergehenden Ansprüche, **dadurch gekennzeichnet, dass** der Absolutdruck im Bereich von 5 bis 50 bar liegt.

27. Verfahren nach einem der fünf vorhergehenden Ansprüche, **dadurch gekennzeichnet, dass** die Katalysatorbelastung, ausgedrückt als Masse an kohlenstoffhaltigen Verbindungen im Zulauf, gerechnet als Methanol, bezogen auf das Katalysatorvolumen und die Zeit, im Bereich von 0,1 bis 2,0 kg/L/h liegt.

28. Verfahren nach einem der Ansprüche 22 bis 27, **dadurch gekennzeichnet, dass** die als Katalysator eingesetzten Formkörper ein Festbett bilden, das zwei, drei, vier oder mehr verschiedene Formkörpertypen enthält, die sich bezüglich ihrer Azidität unterscheiden.

29. Verfahren nach dem vorhergehenden Anspruch, **dadurch gekennzeichnet, dass** die als Katalysator eingesetzten Formkörper ein Festbett bilden, das zwei verschiedene Formkörpertypen enthält, wobei die Anordnung der verschiedenen Formkörpertypen in jeweils einer Schicht erfolgt.

30. Verfahren nach dem vorhergehenden Anspruch, **dadurch gekennzeichnet, dass** der Unterschied des molaren Al/Si-Verhältnisses der beiden Formkörpertypen im Bereich von 2 bis 20 liegt.

31. Verfahren nach einem der Ansprüche 22 bis 30, **dadurch gekennzeichnet, dass** eine Regenerierung des eingesetzten Katalysators durch gezieltes Abbrennen der für die Deaktivierung verantwortlichen Beläge erfolgt.

## Claims

1. A shaped body comprising an aluminosilicate and aluminum oxide, wherein the shaped body has a molar Al/Si ratio in the range from 10 to 30 and an at least bimodal pore distribution for pores having a diameter of greater than 1 nm, with the volume of the pores of the shaped body having a diameter of greater than 10 nm corresponding to at least 40% of the total pore volume of the shaped body.

2. The shaped body according to claim 1 which has a molar Al/Si ratio in the range from 11 to 26.

3. The shaped body according to claim 1 or 2 which has a bimodal pore distribution for pores having a diameter of greater than 1 nm.

4. The shaped body according to any of the preceding claims, wherein the volume of the pores of the shaped body having a diameter of greater than 10 nm corresponds to at least 50% of the total pore volume of the shaped body.

5. The shaped body according to any of the preceding claims which has a BET surface area of ≥ 50 m²/g.

6. The shaped body according to any of claims 1 to 4 which has a BET surface area in the range from 100 to 250 m²/g.

7. The shaped body according to any of the preceding claims which comprises from 0.01 to 1.75% by weight of sodium, from 0.01 to 1.75% by weight of potassium, from 0.01 to 0.35% by weight of titanium and from 0.01 to 0.35% by weight of iron, in each case based on the total weight.

8. The shaped body according to any of the preceding claims which is an extrudate having a length:diameter ratio of ≥ 1.

9. The shaped body according to any of the preceding claims which has a cutting hardness of ≥ 10 newton (N).

10. The shaped body according to any of the preceding claims, wherein the aluminosilicate is a clay.

11. The shaped body according to any of the preceding claims, wherein the aluminosilicate is a kaolin.

12. The shaped body according to any of the preceding claims, wherein the aluminosilicate is kaolinite.

13. The shaped body according to any of the preceding claims, wherein the aluminum oxide is gamma-Al₂O₃.

14. The shaped body according to the preceding claim, wherein aluminum hydroxide and/or aluminum oxide/hydroxide (boehmite) is used as precursor for the gamma-aluminum oxide.

15. The shaped body according to any of the preceding claims, wherein the proportion of aluminum oxide in the shaped body is ≥ 50% by weight.

16. A process for producing a shaped body according to any of the preceding claims, which comprises the steps
(I) preparation of a mixture comprising the aluminosilicate, the aluminum oxide as binder, a pasting agent and a solvent,
(II) mixing and densification of the mixture,
(III) shaping of the densified mixture to give a shaped body,
(IV) drying of the shaped body and
(V) calcination of the dried shaped body.

17. The process according to claim 16, wherein the mixture comprises a pore former in step (I).

18. The process according to claim 16 or 17, where the proportion of aluminosilicate in step (I) is selected so that it is in the range from 1 to 30% by weight in the finished shaped body.

19. The process according to any of claims 16 to 18, wherein the proportion of aluminum oxide in step (I) is selected so that it is at least 50% by weight in the finished shaped body.

20. The process according to any of claims 16 to 19, wherein shaping in (III) is carried out by extrusion.

21. The process according to any of claims 16 to 20, wherein the calcination in (V) is carried out at a temperature in the range from 350 to 750°C for a time in the range from 1 to 24 hours.

22. A process for the continuous preparation of methylamines by reaction of methanol and/or dimethyl ether with ammonia in the presence of a heterogeneous catalyst, wherein shaped bodies according to any of claims 1 to 15 are used as catalyst.

23. The process according to the preceding claim, wherein the feed stream comprises methanol and/or dimethyl ether and ammonia together with monomethylamine and/or dimethylamine and/or trimethylamine.

24. The process according to either of the two preceding claims, wherein the molar N/C ratio in the feed mixture is in the range from 0.6 to 4.0.

25. The process according to any of the three preceding claims, wherein the reaction temperature is in the range from 250 to 500°C.

26. The process according to any of the four preceding claims, wherein the absolute pressure is in the range from 5 to 50 bar.

27. The process according to any of the five preceding claims, wherein the space velocity over the catalyst, expressed as mass of carbon-comprising compounds in the feed, calculated as methanol, per catalyst volume and time, is in the range from 0.1 to 2.0 kg/l/h.

28. The process according to any of claims 22 to 27, wherein the shaped bodies used as catalyst form a fixed bed comprising two, three, four or more different types of shaped bodies which differ in terms of their acidity.

29. The process according to the preceding claim, wherein the shaped bodies used as catalyst form a fixed bed comprising two different types of shaped bodies located in separate zones.

30. The process according to the preceding claim, wherein the difference between the molar Al/Si ratio of the two types of shaped bodies is in the range from 2 to 20.

31. The process according to any of claims 22 to 30, wherein regeneration of the used catalyst is carried out by targeted burning-off of the deposits responsible for deactivation.

## Revendications

1. Corps moulé contenant un aluminosilicate et de l'oxyde d'aluminium, **caractérisé en ce que** le corps moulé présente un rapport molaire Al/Si dans la plage de 10 à 35 et pour les pores ayant un diamètre de plus de 1 nm une distribution au moins bimodale des pores, le volume des pores du corps moulé ayant un diamètre de plus de 10 nm représentant au moins 40 % du volume total de pores du corps moulé.

2. Corps moulé selon la revendication 1, **caractérisé en ce qu'**il présente un rapport molaire Al/Si dans la plage de 11 à 26.

3. Corps moulé selon la revendication 1 ou 2, **caractérisé en ce qu'**il présente pour les pores ayant un diamètre de plus de 1 nm une distribution bimodale des pores.

4. Corps moulé selon l'une quelconque des revendications précédentes, **caractérisé en ce que** le volume des pores du corps moulé ayant un diamètre de plus de 10 nm représente au moins 50 % du volume total de pores du corps moulé.

5. Corps moulé selon l'une quelconque des revendications précédentes, **caractérisé en ce qu'**il présente une surface BET de ≥ 50 m²/g.

6. Corps moulé selon l'une quelconque des revendications 1 à 4, **caractérisé en ce qu'**il présente une surface BET dans la plage de 100 à 250 m²/g.

7. Corps moulé selon l'une quelconque des revendications précédentes, **caractérisé en ce que** le corps moulé contient dans la plage de 0,01 à 1,75 % en poids de sodium, 0,01 à 1,75 % en poids de potassium, 0,01 à 0,35 % en poids de titane et 0,01 à 0,35 % en poids de fer, chaque fois par rapport au poids total.

8. Corps moulé selon l'une quelconque des revendications précédentes, **caractérisé en ce qu'**il s'agit d'un extrudat ayant un rapport longueur : diamètre de ≥ 1.

9. Corps moulé selon l'une quelconque des revendications précédentes, **caractérisé en ce qu'**il présente une dureté à la coupe de ≥ 10 newtons (N).

10. Corps moulé selon l'une quelconque des revendications précédentes, **caractérisé en ce que** l'aluminosilicate consiste en une argile.

11. Corps moulé selon l'une quelconque des revendications précédentes, **caractérisé en ce que** l'aluminosilicate consiste en un kaolin.

12. Corps moulé selon l'une quelconque des revendications précédentes, **caractérisé en ce que** l'aluminosilicate consiste en kaolinite.

13. Corps moulé selon l'une quelconque des revendications précédentes, **caractérisé en ce que** l'oxyde d'aluminium consiste en Al₂O₃-gamma.

14. Corps moulé selon l'une quelconque des revendications précédentes, **caractérisé en ce qu'**on utilise comme précurseur de l'oxyde d'aluminium gamma l'hydroxyde d'aluminium et/ou l'oxyde/hydroxyde d'aluminium (boehmite).

15. Corps moulé selon l'une quelconque des revendications précédentes, **caractérisé en ce que** la teneur en oxyde d'aluminium du corps moulé est ≥ 50 % en poids.

16. Procédé pour la production d'un corps moulé selon l'une quelconque des revendications précédentes, **caractérisé en ce qu'**il comprend les étapes
(I) préparation d'un mélange, contenant l'aluminosilicate, l'oxyde d'aluminium en tant que liant, un agent de mise en pâte et un solvant,
(II) mélangeage et compactage du mélange,
(III) moulage du mélange compacté avec obtention d'un corps moulé,
(IV) séchage du corps moulé et
(V) calcination du corps moulé séché.

17. Procédé selon la revendication 16 **caractérisé en ce que** dans l'étape (I) le mélange contient un agent porogène.

18. Procédé selon la revendication 16 ou 17, **caractérisé en ce que** dans l'étape (I) on choisit la concentration de l'aluminosilicate de manière que dans le corps moulé final elle soit comprise entre 1 et 30 % en poids.

19. Procédé selon l'une quelconque des revendications 16 à 18, **caractérisé en ce que** dans l'étape (I) on choisit la concentration de l'oxyde d'aluminium de manière que dans le corps moulé final elle soit d'au moins 50 % en poids.

20. Procédé selon l'une quelconque des revendications 16 à 19, **caractérisé en ce que** le moulage selon (III) s'effectue par extrusion.

21. Procédé selon l'une quelconque des revendications 16 à 20, **caractérisé en ce que** la calcination selon (V) s'effectue à une température dans la plage de 350 à 750 °C et pendant une durée dans la plage de 1 à 24 h.

22. Procédé pour la production continue de méthylamines par mise en réaction de méthanol et/ou d'éther diméthylique avec de l'ammoniac en présence d'un catalyseur hétérogène, **caractérisé en ce qu'**on utilise comme catalyseur des corps moulés selon l'une quelconque des revendications 1 à 15.

23. Procédé selon la revendication précédente, **caractérisé en ce qu'**en plus de méthanol et/ou d'éther diméthylique et d'ammoniac le courant d'alimentation contient de la monométhylamine et/ou de la diméthylamine et/ou de la triméthylamine.

24. Procédé selon l'une quelconque des deux revendications précédentes, **caractérisé en ce que** le rapport molaire N/C dans le mélange d'alimentation se situe dans la plage de 0,6 à 4,0.

25. Procédé selon l'une quelconque des trois revendications précédentes, **caractérisé en ce que** la température de réaction se situe dans la plage de 250 à 500 °C.

26. Procédé selon l'une quelconque des quatre revendications précédentes, **caractérisé en ce que** la pression absolue se situe dans la plage de 5 à 50 bars.

27. Procédé selon l'une quelconque des cinq revendications précédentes, **caractérisé en ce que** la charge de catalyseur, exprimée en tant que masse de composés carbonés dans le courant d'alimentation, calculée en tant que méthanol, par rapport au volume du catalyseur et au temps, se situe dans la plage de 0,1 à 2,0 kg/l/h.

28. Procédé selon l'une quelconque des revendications 22 à 27, **caractérisé en ce que** les corps moulés utilisés en tant que catalyseur forment un lit fixe qui contient deux, trois, quatre ou plus de quatre types de corps moulés différents, qui diffèrent quant à leur acidité.

29. Procédé selon la revendication précédente, **caractérisé en ce que** les corps moulés utilisés en tant que catalyseur forment un lit fixe qui contient deux types de corps moulés différents, la disposition des différents types de corps moulés s'effectuant dans une couche pour chacun.

30. Procédé selon la revendication précédente, **caractérisé en ce que** la différence du rapport molaire Al/Si des deux types de corps moulés se situe dans la plage de 2 à 20.

31. Procédé selon l'une quelconque des revendications 22 à 30, **caractérisé en ce qu'**une régénération du catalyseur utilisé s'effectue par grillage intentionnel des dépôts responsables de l'inactivation.
